Europäisches Patentamt

European Patent Office

Office européen des brevets

(19)

(11) Publication number: **0 234 796**
**A2**

## (12) EUROPEAN PATENT APPLICATION

(21) Application number: **87301132.4**

(22) Date of filing: **10.02.87**

(51) Int. Cl.⁴: **A 61 F 2/50**

(30) Priority: **11.02.86 GB 8603278   03.11.86 US 926128**

(43) Date of publication of application:
**02.09.87   Bulletin   87/36**

(84) Designated Contracting States:
**AT BE CH DE ES FR GB GR IT LI LU NL SE**

(71) Applicant: **Beane, Thomas Frank**
**P.O. Box 708**
**Concord North Carolina 28025  (US)**

(72) Inventor: **Beane, Thomas Frank**
**P.O. Box 708**
**Concord North Carolina 28025  (US)**

(74) Representative: **Warren, Keith Stanley et al**
**BARON & WARREN 18 South End Kensington**
**London W8 5BU  (GB)**

(54) **Prosthetic limb member and orthopedic stockinette.**

㊐ A prosthetic limb member, and an orthopedic stockinette used in the manufacture of such members, having improved surface finishes in order to impart improved visual appearances and enhanced comfort for a wearer. The limb member has smoothly finished surfaces for contact with the body of a wearer and additionally has a desirable strength to weight ratio, decreasing the relative weight of the member. The stockinette is a circularly knit tube having yarn formed into stitches defining a ground and stitches defining terry pile loops extending from the ground. By such a fabric construction, runs are avoided, bonding of the fabric with the resins used is enhanced, and desirable surface appearances and finishes are attainable.

EP 0 234 796 A2

**Description**

## PROSTHETIC LIMB MEMBER AND ORTHOPEDIC STOCKINETTE

This invention relates to prosthetic limb members and an orthopedic stockinette used in the manufacture of such members. More particularly, this invention relates to such members having improved surface finishes in order to impart improved visual appearances and enhanced comfort for a wearer.

Prior prosthetic limb members have been made using orthopedic stockinette which typically is a circular, plain or rib knit fabric made using cotton or polyester yarns. Such fabrics, while relatively inexpensive, have presented certain problems for both makers and wearers of prosthetic limb members. In particular, plain knit fabrics will run if picked, thereby losing strength and weakening a limb member. The surface finish imparted by the use of rib knit fabrics typically shows the texture of the fabric, lacking the desirable "orange peel" appearance of slight irregularity or non-uniformity which more closely approaches the character of human skin. Where polyester is used as a yarn, the abrasive characteristic of that material precludes obtaining a smooth ground or polished surface where the edge of the stockinette fabric protrudes from the end of an otherwise finished limb, as the exposed fibers form a sharpened array approaching the characteristics of a file.

With the foregoing in mind, it is an object of this invention to aid a maker of prosthetic limb members in attaining a desirable visual appearance and strength for a limb member. In realizing this object of the present invention, a stockinette is provided which is a circularly knit tube having yarn formed into stitches defining a ground and stitches defining terry pile loops extending from the ground. By such a fabric construction, runs are avoided, bonding of the fabric with the resins used is enhanced, and desirable surface appearances and finishes are attainable.

Yet a further object of this invention is to provide a wearer of a prosthetic limb member with a limb member of greater strength and comfort. In realizing this object of the present invention, a limb member is provided which has smoothly finished surfaces for contact with the body of a wearer and additionally has a desirable strength to weight ratio, decreasing the relative weight of the member.

While the present invention will be described more fully hereinafter with reference to a preferred embodiment of the present invention, it is to be understood at the outset of the description which follows that persons of skill in the appropriate arts may modify the invention here described while still achieving the favorable results of this invention. Accordingly, the description which follows is to be understood as being a broad, teaching disclosure directed to persons of skill in the appropriate arts, and not as limiting upon the present invention.

Illustrations of the conventional manner of making an artificial limb member such as an artificial arm or leg may be found in prior patent publications and in prior publications of manuals used in the orthotic and prosthetic field, to which the interested reader is referred. Illustrations of a fabric similar to that described hereinafter may be found in prior Beane United States Patent 4,284,507, Figures 5 through 7, to which the interested reader is referred.

In the orthopedic field, casts are usually formed around broken limbs by first pulling a stockinette over the limb, with felt being wrapped around the stockinette to provide both comfort and bulk. A further stockinette may be pulled over the felt to keep it in place. Plaster is then applied and allowed to set.

Prosthetic limb members are commonly made by first pulling a stockinette over a positive mold in the shape of the limb to be formed. Resin is then applied to the stockinette, and felt wound therearound. Further layers of resin and stockinette are then applied to achieve desired thickness, contours and strength. The stockinettes used may be of successively finer mesh in efforts to improve the finish. The positive mold is removed, and suction applied to the interior of the newly formed member leg to ensure that the resin adequately penetrates the various fabric layers. The resin is then cured to provide a rigid, hollow member formed as a laminar structure of orthopedic stockinette, felt and resins.

The positive mold is usually made of plaster. However, this positive mold may be made from a negative mold, which itself is made by pulling a stockinette over the patient's other limb. After the plaster has set, the negative mold is removed for use in producing the positive mold.

In order to achieve greater strength, comfort and desirable finishes in accordance with this invention, the stockinette used is a circularly knit tube of yarn knit into stitches defining a ground and stitches defining terry pile loops extending from the ground to a predetermined height. In a limb member formed in accordance with this invention, the terry pile loops facilitate bonding with the resins and finishing of the limb member with smooth, visually appealing surfaces. In particular, it has been observed that the pile loops both wick resin throughout the fabric structure more easily than is the case with plain or rib knit fabrics, and also provide both a somewhat random distribution over the surface of a finished limb (so as to impart more of the desired "orange peel" appearance) and a reservoir of material which apparently melts during buffing to achieve an edge surface which is smooth to the touch (so as to lack the file-like character of prior limb members). The enhanced wicking of resins aids in the production of limb members of acceptable strength without necessity of added felt materials, thereby decreasing the weight of the members and contributing to comfort for a wearer.

In accordance with this invention, the tube may be seamless and be made of polyester yarn, knit into courses having from about 8 to about 14 stitches per centimeter and having a diameter, when in relaxed state, of from about 2.5 centimeters to about 30 centimeters. In such a tube, the terry pile loops may have a height of from about 1 centimeter to about 10 centimeters. The terry pile loops may appear on each course or on less than all courses of knitted stitches. The invention also contemplates that the tube may be formed of yarn of a first type knit into the stitches defining terry pile loops and yarn of a second type knit into the stitches defining a ground. If such a multiple yarn fabric is used, then it is preferred that the first type of yarn be polyester for imparting smooth, visually appealing surfaces to the limb member and the second type of yarn be an aramid for imparting strength to the limb member.

Should the stockinette of this invention be used against the skin, it acts as a cushion to achieve good padding and comfort for the wearer. The loops of the fabric also draw excess moisture away, thereby reducing sweat build-up and increasing comfort. The terry pile loop fabric may also be used as a padding to cushion points between braces and a limb and also can be incorporated into support devices such as a girdle.

In the specification there has been set forth a preferred embodiment of the invention and, although specific terms are used, the description thus given uses terminology in a generic and descriptive sense only and not for purposes of limitation.

## Claims

1. A prosthetic limb member formed as a laminar structure of orthopedic stockinette and resins, characterized in that the orthopedic stockinette comprises a circularly knit tube of yarn knit into stitches defining a ground and stitches defining terry pile loops extending from the ground to a predetermined height, the terry pile loops facilitating bonding with the resins and finishing of the limb member with smooth, visually appealing surfaces.

2. An orthopedic stockinette for use in the manufacture of a prosthetic limb member, characterized by being formed as a circularly knit tube of yarn knit into stitches defining a ground and stitches defining terry pile loops extending from the ground to a predetermined height, the terry pile loops facilitating bonding with the resins and finishing of the limb member with smooth, visually appealing surfaces.

3. A limb member or stockinette according to claim 1 or 2, characterized in that the tube comprises polyester yarn.

4. A limb member or stockinette according to claim 1, 2 or 3, characterized in that the tube comprises yarn knit into courses having from about 8 to about 14 stitches per centimeter.

5. A limb member or stockinette according to any preceding claim, characterized in that the tube comprises yarn knit into terry pile loops having a height of from about 1 centimeter to about 10 centimeters.

6. A limb member of stockinette according to any preceding claim, characterized in that the tube has a diameter, when in relaxed state, of from about 2.5 centimeters to about 30 centimeters.

7. A limb member or stockinette according to any preceding claim, characterized in that the tube is seamless.

8. A limb member or stockinette according to any preceding claim characterized in that the tube comprises yarn of a first type knit into said stitches defining terry pile loops and yarn of a second type knit into said stitches defining a ground.

9. A limb member or stockinette according to claim 8, characterized in that the first type of yarn is polyester for imparting smooth, visually appealing surfaces to said limb member and said second type of yarn is an aramid for imparting strength to said limb member.

10. A method of making a prosthetic limb member in which orthopedic stockinette and resins are laminated together, characterized by the step of providing an orthopedic stockinette in the form of a circularly knit tube of yarn knit into stitches defining a ground and stitches defining terry pile loops extending from the ground to a predetermined height, the terry pile loops facilitating bonding with the resins and finishing of the limb member with smooth, visually appealing surfaces.

11. A method according to claim 10, characterized in that the orthopedic stockinette is in the form of a seamless, circularly knit tube having a diameter, when in relaxed state, of from about 2.5 centimeters to about 30 centimeters, the tube being formed of yarn knit in courses having from about 8 to about 14 stitches per centimeter and into stitches defining a ground and stitches defining terry pile loops extending from the ground to a height of from about 1 centimeter to about 10 centimeters.